# EUROPÄISCHE PATENTANMELDUNG

(11) **EP 2 229 915 A2**
(43) Veröffentlichungstag der Anmeldung: **22.09.2010**
(21) Anmeldenummer: 10002559.2
(22) Anmeldetag: 11.03.2010
(51) Int. Cl.: A61C 17/08, A61M 1/00

(54) **Sauginstrument**

(30) Priorität: 21.03.2009 DE 102009013836
(71) Anmelder: Dürr Dental AG, 74321 Bietigheim-Bissingen (DE)
(72) Erfinder: Nonnenmacher, Eberhardt, 74379 Ingersheim (DE)
(74) Vertreter: Ostertag, Reinhard

(57) **Zusammenfassung**

Ein Sauginstrument für chirurgische Zwecke umfasst ein rohrförmiges Anschlussstück und eine auf dessen Arbeitsende aufsetzbare Saugspitze (10). Diese hat einen schlanken kegelförmigen Hauptabschnitt (12) und einen im Wesentlichen zylindrischen Verbindungsabschnitt (14). Der Verbindungsabschnitt (14) hat zwei einander gegenüberliegende Abplattungen (22), die zusammen mit Ausnehmungen (24) im Basisbereich des Hauptabschnittes (12) eine Nebenluftnut vorgeben, die zusammen mit der Innenfläche des Anschlussstückes (16) jeweils einen Nebenluftkanal bilden. Die durch die Nebenluftkanäle strömende Nebenluft bildet zugleich eine Strahlpumpe.

## Beschreibung

Die Erfindung betrifft ein Sauginstrument gemäß dem Oberbegriff des Anspruches 1.

Die DE 10 2007 006 613 A1 offenbart eine Saugkanüle für eine dentale Saugeinrichtung, welche an ihrem mit einem Anschlussstück verbindbaren Ende nach außen offene Nuten aufweist, über welche Nebenluft angesaugt werden kann, wenn die am freien Ende der Saugkanüle liegende Saugöffnung verschlossen ist, z. B. durch dichtes Anliegen an einem weichen Gewebe.

Derartige bekannte Saugkanülen finden im dentalen Bereich Verwendung und haben verhältnismäßig großen freien Querschnitt, um auch größere Wassermengen und größere Sprühnebelmengen rasch aus dem Mund eines Patienten abzusaugen.

Für chirurgische Zwecke sind derartige Saugkanülen zu sperrig. Es werden dort Sauginstrumente verwendet, die sehr viel kleineren Außendurchmesser und sehr viel kleineren lichten Querschnitt aufweisen. Diese Sauginstrumente sind über verhältnismäßig lange Schläuche ebenfalls geringem Querschnittes mit einer Unterdruckquelle verbunden.

Herkömmliche Unterdruckquellen können nur einen begrenzten Saugdruck bereitstellen, der z. B. bei mittelgroßen Sauganlagen im Bereich von 140 mmWs liegt.

Das bei Operationen abgesaugte Blut hat schon eine deutlich höhere Viskosität als Wasser, und bei Operationen müssen auch Blutgerinsel und kleinere Gewebestücke mit abgesaugt werden. Hierbei kann es leicht zu einem Verstopfen des Sauginstrumentes kommen, so dass dieses gereinigt oder ausgetauscht werden muss. Beides verzögert die eigentlich durchzuführende Arbeit.

Viele dieser in der Praxis auftretenden Verstopfungen von Sauginstrumenten ließen lich dadurch beheben, dass man den Unterdruck erhöht. Bei schon existierenden Sauganlagen würde dies aber kostspielige neue Investitionen bedeuten.

Durch die vorliegende Erfindung soll daher ein Sauginstrument gemäß dem Oberbegriff des Anspruches 1 so weitergebildet werden, dass an seiner Arbeitsöffnung ein höherer Unterdruck bereitgestellt wird.

Diese Aufgabe ist erfindungsgemäß gelöst durch ein Sauginstrument mit den im Anspruch 1 angegebenen Merkmalen.

Die erfindungsgemäße Lösung nutzt aus, dass bei Sauginstrumenten der eingangs beschriebenen Art ein Nebenluftstrom angesaugt wird, der dazu dient, ein Rückfließen von Flüssigkeit zu verhindern. Erfindungsgemäß wird dieser Nebenluftstrom nun zusätzlich dazu verwendet, nach Art einer Strahlpumpe den Unterdruck zu erhöhen, der in der Saugspitze des Sauginstrumentes herrscht. Dies erfolgt über eine spezielle Bemessung der Querschnittsflächen der Nebenluftkanäle, wie sie im Anspruch 1 angegeben ist.

Die Nebenluftkanäle sind begrenzt durch Nebenluftnuten, die in einer Kontaktfläche der Saugspitze oder des Anschlussstückes vorgesehen sind, und die gegenüberliegende Kontaktfläche von Anschlussstück oder Saugspitze. Dabei soll unter Nut jede Oberflächengeometrie verstanden, die von einer nutfreien Grundkontaktfläche zurückspringt, also insbesondere auch Abplattungen. Letztlich geht es darum, dass die Kontaktflächen, über welche Saugspitze und Anschlussstück in Berrührung stehen nicht exakt komplementär sind, vielmehr zusammen flache Nebenlufkanäle begrenzen.

Vorteilhafte Weiterbildungen der Erfindung sind in Unteransprüchen angegeben.

Querschnittsverhältnisse, wie sie im Anspruch 2 angegeben sind, führen bei üblichen Abmessungen von chirurgischen Sauginstrumenten zu einer besonders guten Unterdruckerhöhung am Arbeitsende des Sauginstrumentes.

Mit der Weiterbildung der Erfindung gemäß Anspruch 3 wird die Nebenluft besonders strömungsgünstig angesaugt. Man erhält daher hohe Strömungsgeschwindigkeiten der Nebenluft, was die Strahl-Pumpwirkung verbessert.

Bei einem Sauginstrument gemäß Anspruch 4 ist gewährleistet, dass die Nebenluft nicht aus der unmittelbaren Nachbarschaft des freien Endes der Saugspitze angesaugt wird. Dort könnten Luftströmungen stören.

Die Weiterbildung der Erfindung gemäß Anspruch 5 gestattet es, auf besonders einfache Weise die Saugspitze und das Anschlussstück zu verbinden und in diese Verbindung die Nebenluftkanäle zu integrieren.

Bei Berücksichtigung der im Anspruch 6 angegebenen Tiefe der Nebenluftnuten erhält man wieder eine besonders gute Unterdruckerhöhung.

Auch die Weiterbildung der Erfindung gemäß Anspruch 7 dient der Verstärkung der Unterdrucküberhöhung.

Die Weiterbildung der Erfindung gemäß Anspruch 8 ist im Hinblick auf guten Blickkontakt zur Absaugstelle und im Hinblick auf eine ggfs. erwünschte Abdichtung der Saugspitze beim Aufsetzen auf Gewebe von Vorteil.

Dabei haben sich Öffnungswinkel der kegelförmigen Mantelfläche der Saugspitze, wie sie im Anspruch 9 angegeben sind, als besonders vorteilhaft erwiesen.

Die Weiterbildung der Erfindung gemäß Anspruch 10 ist in verschiedener Hinsicht vorteilhaft: Zum Einen sind die Oberflächen von glatt gespritzten Polypropylen-Werkstücken so glatt, dass sich dort Verunreinigungen schlecht festsetzen können. Zum Anderen hat das Polypropylen eine gewisse Elastizität, so dass man durch Verformung des freien Endes der Saugspitze Dichtstellen zu unebenen Gewebebereichen herstellen kann. Außerdem lassen sich Polypropylen-Werkstücke leicht in der für medizinische Zwecke erforderlichen hygienischen Qualität herstellen.

Die Bemessungsangaben der Ansprüche 11 und 12 führen zu einem Sauginstrument, welches sich besonders gut für feine Absaugaufgaben in der Chirurgie eignet.

Die Weiterbildung der Erfindung gemäß Anspruch 13 gestattet es, den Nebenluftstrom zu steuern.

Dabei ist die Steuerung durch eine Drehbewegung besonders einfach und genau zu realisieren. Eine solche ist Gegenstand des Anspruches 14.

Bei einem Sauginstrument gemäß Anspruch 15 kann man den Nebenluftstrom ganz unterbinden.

Die Weiterbildung der Erfindung gemäß Anspruch 16 ist im Hinblick auf eine von Fertigungsschwankungen weitgehend unabhängige Abdichtung der Nebenluftnuten in der Schließstellung des Steuerelementes von Vorteil.

Mit der Weiterbildung der Erfindung gemäß Anspruch 17 wird erreicht, dass die Ränder der Steuerabschnitte linienhaft mit den Materialoberflächen zusammenarbeiten, welche die Eintrittsenden der Nebenluftkanäle umgeben.

Nachstehend wird die Erfindung anhand eines Ausführungsbeispieles unter Bezugnahme auf die Zeichnung näher erläutert. In dieser zeigen:
- Figur 1: eine perspektivische Ansicht einer Saug- spitze eines chirurgischen Sauginstrumentes;
- Figur 2: eine seitliche Ansicht eines abgewinkelten rohrförmigen Anschlussstückes, auf welches die Saugspitze nach Figu1 aufsetzbar ist;
- Figur 3: eine Aufsicht auf die Saugspitze nach Figur 1 gesehen von deren freiem Arbeitsende her;
- Figur 4: eine seitliche Ansicht der Saugspitze nach Figur 1;
- Figur 5: einen axialen Schnitt durch die Saugspitze nach Figur 4 längs der dortigen Schnittlinie A-A;
- Figur 6: eine vergrößerte Detailansicht des einlasssei- tigen Endes einer Nebenlufnut der Saugspitze nach Figur 5;
- Figur 7: einen axialen Schnitt durch ein abgewandeltes Sauginstrument, welches mit einem Drehschieber- ventil zur Steuerung des Nebenluftstroms verse- hen ist, gezeigt in der Schließstellung;
- Figur 8: einen ähnlichen Schnitt wie Figur 7, in welchem jedoch das Drehschieberventil in der Offenstel- lung wiedergegeben ist;
- Figur 9: eine seitliche Ansicht einer Steuerhülse des Drehschieberventiles nach den Figuren 7 und 8;
- Figur 10: einen axialen Schnitt durch die Steuerhülse nach Figur 10; und
- Figur 11: eine Stirnansicht der Steuerhülse nach den Figuren 9 und 10.

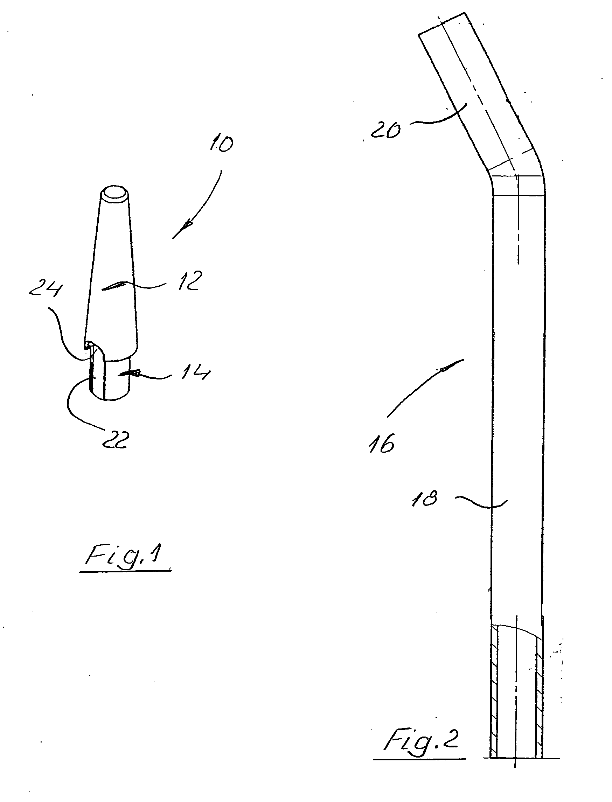

Figur 1 zeigt eine Saugspitze 10, welche einen schlank kegelförmigen Hauptabschnitt 12 sowie einen im Wesentlichen zylindrischen Verbindungsabschnitt 14 aufweist.

Die Saugspitze 10 ist zur Anbringung an einem rohrförmigen Anschlussstück 16 bestimmt, wie es in Figur 2 gezeigt ist. Das Anschlussstück 16 besteht aus zwei Rohrabschnitten 18, 20, wovon der in Figur 2 untere Rohrabschnitt 18 etwa 3/4, der in Figur 2 obere Rohrabschnitt 20 etwa 1/4 der Gesamtlänge ausmacht. Der Rohrabschnitt 20 ist zur Achse des Rohrabschnittes 18 um etwa 25° verkippt.

Durch diese Verkippung erhält ein Sauginstrument, welches man durch Aufstecken der Saugspitze 10 auf das Anschlussstück 16 erhält, eine ergonomisch günstige Form.

Unter Arbeitsbedingungen sitzt die Saugspitze 10 im Presssitz im Rohrabschnitt 20, wozu eine entsprechende Presspassung zwischen dem Innendurchmesser des Rohrabschnittes 20 und dem Außendurchmesser des Verbindungsabschnittes 14 vorgesehen ist.

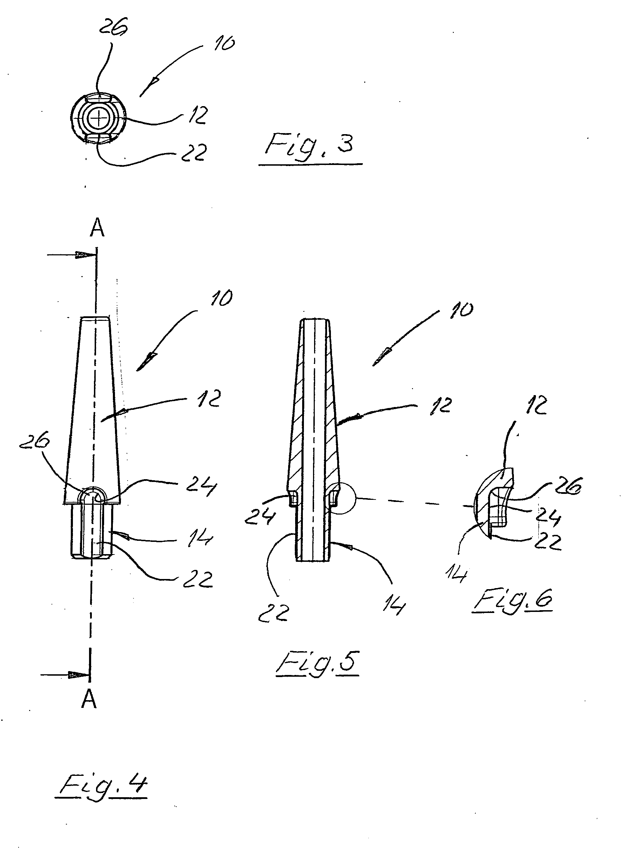

Die Saugspitze 10 trägt an ihrem Verbindungsabschnitt 14 zwei einander diametral gegenüberliegende Abplattungen 22, die sich jeweils in eine Ausnehmung 24 fortsetzen, die im Bereich der zwischen dem Hauptabschnitt 12 und dem Verbindungsabschnitt 14 gebildeten Schulter der Saugspitze liegt. Die Ausnehmungen 24 haben einen gebogenen Boden 26 (Figur 6), dessen eines Ende fluchtend in die benachbarte Abplattung 22 übergeht und dessen anderes Ende in einer zur Achse der Saugspitze 10 transversalen Ebene liegt.

Steckt man die Saugspitze 10 in den Rohrabschnitt 20, so begrenzen die Abplattungen 22 zusammen mit der Innenfläche des Rohrabschnittes 20 Nebenluftkanäle mit einer Querschnittsform, die einem niederen Kreisabschnitt entspricht, wobei die Höhe des entsprechenden Kreissegmentes typischerweise 0,2 bis 0,4 mm, vorzugsweise 0,3 mm betragen kann.

Wünscht man Nebenluftkanäle mit etwas größerer Tiefe, so kann man die Abplattungen 22 durch flache Nuten ersetzen, die in dem dann etwas dicker ausgeführten Verbindungsabschnitt 14 vorgesehen sind. In der vorliegenden Beschreibung und den Ansprüchen sollen die Abplattungen als Grenzfall einer Nut mit verschwindender Seitenwand angesehen werden.

Wünscht man den Querschnitt eines Nebenluftkanales bei gleicher Tiefe (radiale Erstreckung) zu vergrößern, so kann man die Umfangserstreckung der Abplattungen 22 größer wählen. Auch kann man die Anzahl der Abplattungen 22 vergrößern bis hin zu dem Fall, dass nur noch kleine teilzylindrische Umfangsbereiche des Verbindungsabschnittes 14 verbleiben, dessen Querschnitt dann einem regelmäßigen Polygon mit abgerundeten Ecken ähnelt.

Die Saugspitze 10 ist ein aus thermoplastischem Material hergestelltes Spritzteil. Bevorzugt findet Polypropylen Verwendung. Der lichte Innendurchmesser der Saugspitze 10 ist durch die Saugerfordernisse vorgegeben.

Die Wandstärke des Verbindungsabschnittes 14 wird im Hinblick auf eine sichere Presspassung im Rohrabschnitt 20 und im Hinblick auf eine an der Stoßstelle zur Innenfläche Rohrabschnittes gewünschte abrupte Querschnittserweiterung gewählt, die die Strahlpumpenwirkung beeinflusst.

Die Dicke der Wand des Hauptabschnittes 12 der Saugspitze 10 wird so gewählt, dass man beim in der Zeichnung oben liegenden freien Rand der Saugspitze eine Wandstärke hat, die folgende Bedingungen erfüllt: Der freie Rand der Saugspitze darf keine Schneide bilden. Der freie Rand der Saugspitze soll elastisch geringfügig verformbar sein, um sich Unebenheiten an Hart- oder Weichgeweben etwas anpassen zu können. Der Öffnungswinkel des Kegels der Außenfläche der Saugspitze 10 soll möglichst klein sein, um einen guten Sichtkontakt zur Arbeitsstelle zu gewährleisten und auch ein Arbeiten dicht an Hindernissen zu ermöglichen. Andererseits soll die Schulter, die beim in der Zeichnung unteren Ende des Hauptabschnittes 12 liegt, ausreichend hoch sein, um für den gebogenen Boden 26 noch eine Radialerstreckung zu ermöglichen.

Beim Anschlussstück 16 handelt es sich vorzugsweise um ein Kunststoffspritzteil aus PVC.

Die Arbeitsweise eines Sauginstrumentes, welches man durch Aufsetzen einer Saugspitze 10 auf ein Anschlussstück 16 erhält, ist folgende:
Eine an das in Figur 2 unten liegende Ende des Anschlussstückes 16 angeschlossene Unterdruckquelle saugt Luft vom freien Ende der Saugspitze 10 her an. Befinden sich vor der Saugspitze Flüssigkeiten oder kleinere Feststoffpartikel, werden diese über die Saugspitze und das Anschlussstück 16 abgesaugt und einem Abscheider zugeführt, der zwischen das Sauginstrument und die Unterdruckquelle geschaltet is

Gleichzeitig wird ein Nebenluftstrom über die Nebenluftkanäle angesaugt, welche durch die Abplattungen 22 und die Innenfläche des Rohrabschnittes 20 vorgegeben sind.

Dieser verhindert ein Rückfließen von Flüssigkeit,
Wie aus der Zeichnung ersichtlich und aus der obenstehenden Beschreibung erkennbar, ist die Gesamtquerschnittsfläche der Nebenluftkanäle deutlich geringer als die Querschnittsfläche der am Ende der Saugspitze 10 liegenden Saugöffnung. In der Praxis ist ein Verhältnis von 10% gut geeignet. Im Hinblick auf die Erfüllung anderer Anforderungen sind aber auch Querschnittsverhältnisse bis zu 20% oder 33% geeignet.

Dieser Nebenluftstrom reicht dazu aus, ein Rückfließen angesaugter Flüssigkeit im Sauginstrument zu verhindern.

Wird die Saugöffnung der Saugspitze 10 durch hochviskose Flüssigkeit oder ein geronnenes Blutvolumen verschlossen, so wirkt hierauf ein erhöhter Unterdruck, der zum Teil dadurch erhalten wird, dass die über die Nebenluftkanäle achsparallel ins Innere des Anschlussstückes 16 gezogene Nebenluft nach Art einer Wasserstrahlpumpe eine Unterdruckerhöhung bringt. Diese kann in der Praxis 5 bis 10 % des Druckes ausmachen, den die Unterdruckquelle selbst erbringt.

Durch diese Erhöhung des Unterdruckes ist es dann in vielen Fällen möglich, die Verstopfung abzusaugen, und gelangt das verstopfende Material dann in den Bereich der bei den in der Zeichnung unten liegenden Enden der Abplattungen 22 liegenden Strahlpumpe, werden die verstopfenden Materialvolumina etwas aufgerissen, so dass sie dann sicher durch das Anschlussstück 16 und einen sich hieran anschließenden Schlauch in den Abscheider gefördert werden.

Bei den oben für die Saugspitze 10 und das Anschlussstück 16 angegebenen Materialien können z. B. folgende Geometrien verwendet werden:

| | |
|---|---|
| Innendurchmesser der Rohrabschnitte 18, 20 | 4,9 mm |
| Wandstärke der Rohrabschnitte 18, 20 | 0,75 mm |
| Gesamtlänge des Anschlussstückes 16 | 155 mm |
| Gesamtlänge der Saugspitze 10 | 31 mm |
| Länge des Verbindungsabschnitts 14 | 7 mm |
| axiale Erstreckung des Bodens 26 | 2 mm |
| Innendurchmesser der Saugspitze | 2,75 mm |
| Öffnungskegel der Mantelfläche der Saugspitze | 8° |
| Außendurchmesser des Verbindungsabschnittes | 5 mm |
| größter Außenmesser des Hauptabschnittes 12 | 6,8 mm |

Aus den oben angegebenen Abmessungsdaten ist erkennbar, dass sich der Querschnitt des Förderweges beim in Strömungsrichtung hinteren Endes der Saugspitze schlagartig von etwas weniger als 3 mm auf rund 5 mm erweitert. An der gleichen Stelle treten die Nebenluftstrahlen in den Rohrabschnitt 20 ein. Man erhält dort so eine gute Strahlpumpenwirkung.

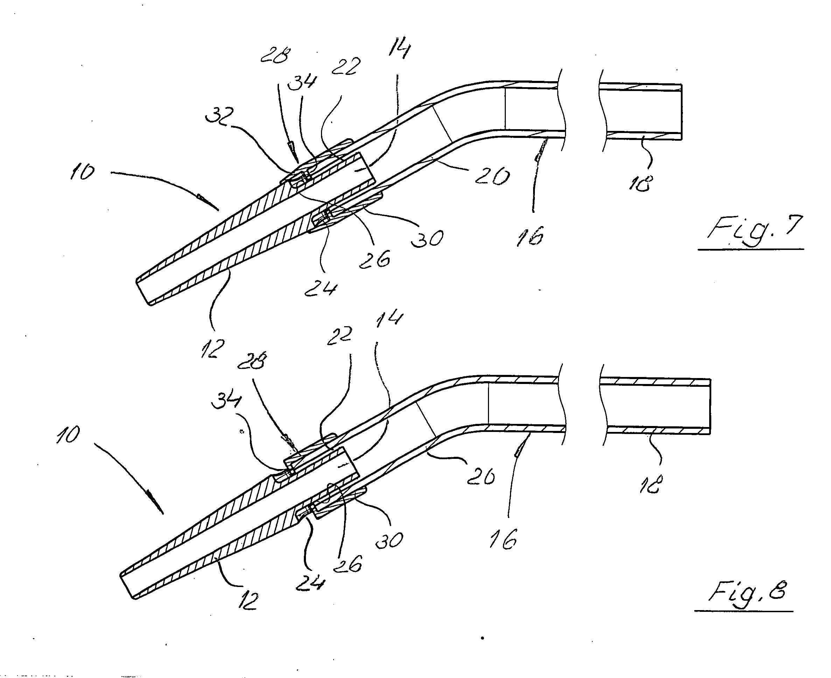

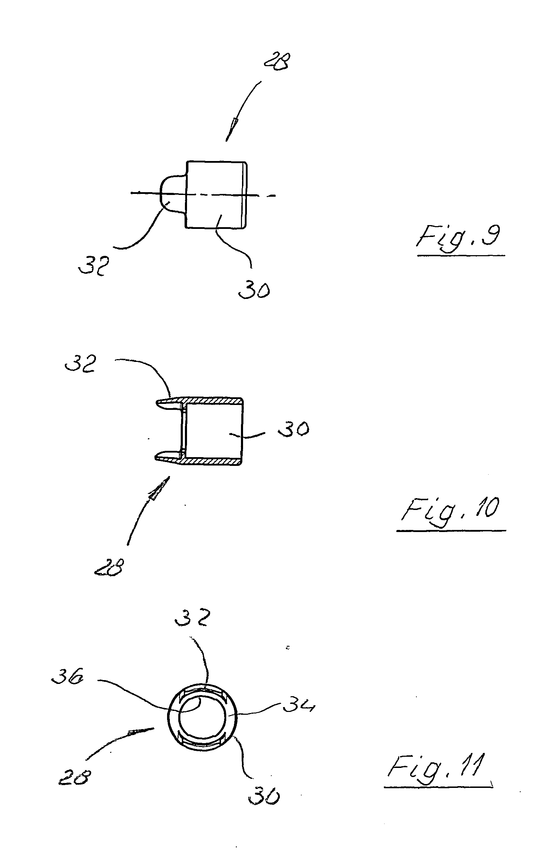

Die Figuren 7 bis 10 zeigen ein abgewandeltes Ausführungsbeispiel eines chirurgischen Sauginstrumentes, bei welchem die Möglichkeit besteht, den Durchsatz an Nebenluft zu steuern. Komponenten, die vorstehend schon unter Bezugnahme auf die Figuren 1 bis 6 erläutert wurden, sind wieder mit denselben Bezugszeichen versehen und brauchen nachstehend nicht nochmals detailliert beschrieben zu werden.

Bei dem Sauginstrument nach den Figuren 7 und 8 ist zwischen die Saugspitze 10 und das Anschlussstück 16 eine Steuerhülse 28 eingefügt.

Die Steuerhülse 28 hat einen hinten liegenden Hülsenabschnitt 30, der drehbar auf den Rohrabschnitt 20 des Anschlussstückes 16 aufgesetzt ist. An den Hülsenabschnitt 30 sind zwei einander diametral gegenüberliegende teilkegelstumpfförmige Steuerflügel 32 angeformt, die zwischen einer die Ausnehmungen 24 der Nebenluftnuten überdeckenden Schließstellung und einer die Ausnehmungen 24 freigebenden Offenstellung bewegbar sind, in dem man die Steuerhülse 28 um ihre Achse dreht.

Wie aus den Zeichnungen ersichtlich, nimmt die Wandstärke der Steuerflügel 32 vom Hülsenabschnitt 30 zum freien Ende hin ab, und der Kegel, auf welchem die Außenflächen der Steuerflügel 32 liegen, hat etwas größeren Öffnungswinkel als der Kegel, der durch die Außenfläche des Hauptabschnittes 12 der Saugspitze 10 vorgegeben ist. Bei praktischem Ausführungsbeispiel kann der Öffnungswinkel des Kegels der Steuerflügel 32 um 5 bis 10° größer sein als der Öffnungswinkel der Außenfläche des Hauptabschnittes 12.

Die Randkontur der Steuerflügel 32 ist in radialer Richtung gesehen größer als die Randkontur der Ausnehmungen 24, so dass die Steuerflügel 32 die Ausnehmungen 24 voll überdecken und verschließen können.

In Zwischenstellungen der Steuerhülse 28 zwischen der vollen Offenstellung und der Schließstellung erhält man Nebenluftströme entsprechend reduzierter Größe.

Um die Steuerhülse 28 in axialer Richtung bezüglich der Saugspitze 10 und des Anschlussstückes 16 zu positionieren, hat die Steuerhülse 28 an der Übergangsstelle zwischen dem Hülsenabschnitt 30 und den Steuerflügeln 32 einen radial nach innen kragenden Positionierflansch 34.

Dieser stellt über den größten Teil seines Umfanges eine glatte Fortsetzung der Innenfläche des Rohrabschnittes 20 dar. An zwei einander diametral gegenüberliegenden Stellen weist der innere Rand des Positionierflansches 34 zwei nach innen weiter vorstehende Vorsprünge 36 auf, die bis zum Boden der Ausnehmung 24 bzw. bis zu den Abplattungen 22 reicht. Auf diese Weise wird eine weitere Dichtstelle in der Schließstellung des durch die Steuerhülse 28 und die Ausnehmungen 24 gebildeten Drehschieberventiles erhalten.

Da dieser Vorsprung, wie aus der Zeichnung ersichtlich, sehr klein ist, kann er beim Verdrehen der Steuerhülse 28 bezüglich der Saugspitze 10 etwas elastisch verformt werden. Man erhält auf diese Weise zugleich auch eine taktil wahrnehmbare Markierung der Schließstellung des Drehschieberventiles.

Man erkennt, dass man die Steuerhülse 28 wahlweise verwenden kann, wie in den Figuren 7 bis 11 dargestellt, sie aber auch weglassen kann und die gleiche Saugspitze 10 mit dem gleichen Anschlussstück 16 auch als Sauginstrument ohne integriertes Drehschieberventil verwenden kann. In diesem Falle kann man eine vorübergehende Drosselung des Nebenluftstromes dadurch vornehmen, dass man eine oder beide der Ausnehmungen 24 ganz oder teilweise mit einem Finger verschließt.

## Patentansprüche

1. Sauginstrument umfassend ein mit einer Unterdruckquelle verbindbares Anschlussstück (16) und eine auf letzteres aufgesetzte Saugspitze (10), wobei Anschlussstück (16) und Saugspitze (10) über komplementäre Kontaktflächen im Wesentlichen formschlüssig zusammenarbeiten, wobei in mindestens einer der Kontaktfläche eine Nebenluftnut (22, 24) vorgesehen ist, die zur anderen der Kontaktflächen hin offen ist, wodurch ein Nebenluftkanal erhalten wird, **dadurch gekennzeichnet, dass** der Gesamtquerschnitt aller Nebenluftkanäle kleiner ist als ein Drittel des freien Querschnittes der Saugspitze (10).

2. Sauginstrument nach Anspruch 1, **dadurch gekennzeichnet, dass** der Gesamtquerschnitt aller Nebenluftkanäle kleiner ist als ein Fünftel, vorzugsweise kleiner als etwa ein Zehntel des lichten Querschnittes der Saugspitze (10).

3. Sauginstrument nach Anspruch 1 oder 2, **dadurch gekennzeichnet, dass** ein einlassseitiger Abschnitt des Bodens (26) der Nebenluftnut (22, 24) eine radiale Erstreckungskomponente hat.

4. Sauginstrument nach Anspruch 3, **dadurch gekennzeichnet, dass** ein freier äußerer Abschnitt des Bodens (26) der Nebenluftnut (22, 24) in einer senkrecht auf der Achse der Saugspitze (10) liegenden Ebene liegt.

5. Sauginstrument nach einem der Ansprüche 1 bis 4,
**dadurch gekennzeichnet, dass** die Nebenluftnut (22, 24) zumindest teilweise durch eine Abplattung an einem im Wesentlichen zylindrischen Verbindungsabschnitt (14) der Saugspitze (10) gebildet ist.

6. Sauginstrument nach einem der Ansprüche 1 bis 5,
**dadurch gekennzeichnet, dass** der maximale Abstand des tiefsten Punktes der Nebenluftnut (22, 24) von der gegenüberliegenden Kontaktfläche etwa 0,2 bis 0,4 mm, vorzugsweise etwa 0,3 mm beträgt.

7. Sauginstrument nach einem der Ansprüche 1 bis 6,
**gekennzeichnet durch** eine Mehrzahl in Umfangsrichtung verteilter Nebenluftnuten (22, 24), die vorzugsweise in Umfangsrichtung äquidistant angeordnet sind.

8. Sauginstrument nach einem der Ansprüche 1 bis 7,
**dadurch gekennzeichnet, dass** die Saugspitze (10) eine kegelförmige Mantelfläche hat.

9. Sauginstrument nach Anspruch 8, **dadurch gekennzeichnet, dass** der Öffnungwinkel der kegelförmigen Mantelfläche zwischen 5 und 15°, vorzugsweise etwa 8° beträgt.

10. Sauginstrument nach einem der Ansprüche 1 bis 9,
**dadurch gekennzeichnet, dass** die Saugspitze (10) aus Polypropylen gefertigt ist.

11. Sauginstrument nach einem der Ansprüche 1 bis 10,
**dadurch gekennzeichnet, dass** die Saugspitze (10) einen lichten Durchmesser von 2 bis 6 mm, vorzugsweise etwa 3 mm aufweist, wobei vorzugsweise der lichte Durchmesser des Anschlussstückes (20) 2 bis 4mm größer ist, vorzugsweise etwa 3 mm größer ist.

12. Sauginstrument nach einem der Ansprüche 1 bis 11,
**dadurch gekennzeichnet, dass** die Saugspitze (10) eine axiale Abmessung von etwa 25 bis 35 mm, vorzugsweise etwa 30 mm aufweist.

13. Sauginstrument nach einem der Ansprüche 1 bis 12,
**gekennzeichnet durch** mindestens ein Steuerelement (28), welches den Luftsatz **durch** mindestens einen der Nebenluftkanäle steuert.

14. Sauginstrument nach Anspruch 13, **dadurch gekennzeichnet, dass** das Steuerelement (28) einen Hülsenabschnitt aufweist, der drehbar auf das Anschlussstück (16) und/oder die Saugspitze (10) aufgesetzt ist und mindestens einen vom Hülsenabschnitt (30) abgelegenen Steuerabschnitt (32) aufweist, der mit einer Nebenluftnut (22, 24) zusammenarbeitet.

15. Sauginstrument nach Anspruch 14, **dadurch gekennzeichnet, dass** der Hülsenabschnitt (30) für jeden Nebenluftkanal einen Steuerabschnitt (32) trägt.

16. Sauginstrument nach Anspruch 14 oder 15, **dadurch**
**gekennzeichnet, dass** die Steuerabschnitte (32) elastisch verformbares Material umfassen und Dichtkanten aufweisen, die mit der Außenfläche der Saugspitze (10) zusammenarbeiten.

17. Sauginstrument nach einem der Ansprüche 14 bis 16,
**dadurch gekennzeichnet, dass** die Steuerabschnitte (32) keilkegelstumpfförmig sind und der Öffnungswinkel des durch sie vorgegebenen Kegels etwas, vorzugsweise etwa 5° bis etwa 10° größer ist als der Öffnungswinkel der kegeligen Außenfläche der Saugspitze (10).
